# EUROPEAN PATENT APPLICATION

(11) **EP 2 915 480 A1**
(43) Date of publication of application: **09.09.2015**
(21) Application number: 14176352.4
(22) Date of filing: 09.07.2014
(51) Int. Cl.: A61B 5/0408

(54) **Sensor module for simultaneously measuring ECG and pulse signal**

(30) Priority: 06.03.2014 US 201414199707
(71) Applicant: MedSense Inc., 30273 Zhubei City, Hsinchu County (TW)
(72) Inventor: Lai, Yu Chen, 326 Yangmei City, Taoyuan County (TW); Chiu, You-Ming, 302 Zhubei City, Hsinchu County (TW)
(74) Representative: 2K Patentanwälte Blasberg Kewitz & Reichel

(57) **Abstract**

The present disclosure provides a sensor module (10a-d, 10) for simultaneously measuring electrocardiography (ECG) and pulse signal of an object, including: a first electrode (12) having a first surface (121) and an opposite, second surface (122); a deformable contact sensor (14) having a first surface (141) and an opposite, second surface (142), wherein the first surfaces (121, 141) of the first electrode (12) and the deformable contact sensor (14) are configured to face toward a region (20) to be measured of the object; a compressible material (16) disposed on at least one of the second surfaces (122, 142) of the first electrode (12) and the deformable contact sensor (14); and a second electrode (18) operatively connected to the first electrode (12).

## Description

### CROSS REFERENCE TO RELATED APPILCATION

This Application claims priority of US Application No. 14/199,707, filed on March 6, 2014, the entirety of which is incorporated by reference herein.

### BACKGROUND OF THE INVENTION

### FIELD OF THE INVENTION

The present invention relates to a sensor module, and in particular relates to a sensor module for measuring electrocardiography (ECG) and pulse signal simultaneously.

### DESCRIPTION OF THE RELATED ART

Through bio-signal self-measurement manners, patients can monitor their own physiology status anytime, to relieve strain on hospital resources and provide needed medical attention to patients. Currently, there are many medical applications to get electrocardiography (ECG) and artery pulse simultaneously. In general, to get artery (radial artery), users need to feel the pulse and have the sensor contacted with the correct position. Traditionally, the ECG electrode and pulse sensor are separately installed, and the sensor must be either fastened by a strap or stuck with a stamp, so users may be free to attach ECG electrodes afterwards. However, this is a tedious procedure.

Therefore, it would be highly desirable to provide a sensor device that may be operated without a fasten means to measure electrocardiography (ECG) and pulse signal simultaneously is desired.

### BRIEF SUMMARY OF THE INVENTION

In one embodiment, the present disclosure provides a sensor module for simultaneously measuring electrocardiography (ECG) and pulse signal of an object, including: a first electrode having a first surface and an opposite, second surface; a deformable contact sensor having a first surface and an opposite, second surface, wherein the first surfaces of the first electrode and the deformable contact sensor are configured to face toward a region to be measured of the object; a compressible material disposed on at least one of the second surfaces of the first electrode and the deformable contact sensor; and a second electrode operatively connected to the first electrode.

In another embodiment, the present disclosure also provides a method of using the sensor module as set forth above, including placing the sensor module on the region to be measured of the object and positioning the sensor module by a hand and touching the second electrode with at least one finger to measure the electrocardiography (ECG) and pulse signal simultaneously.

In still another embodiment, the present disclosure further provides use of a sensor module as set forth above to measure pulse transition time (PTT) and derived indices thereof by calculating an arrival time difference between the ECG and the pulse signal.

A detailed description is given in the following embodiments with reference to the accompanying drawings.

### BRIEF DESCRIPTION OF THE DRAWINGS

The present invention can be more fully understood by reading the subsequent detailed description and examples with references made to the accompanying drawings, wherein:
Fig. 1A is a cross section of a sensor module according to a first embodiment of the present disclosure.
Fig. 1B-1C are cross-sectional views of the sensor module according to the first embodiment of the present disclosure during the measurement.
Fig. 2A is a cross section of a sensor module according to a second embodiment of the present disclosure.
Fig. 2B-2C are cross-sectional views of the sensor module according to the second embodiment of the present disclosure during the measurement.
Fig. 3A and Fig. 4A are cross sections of a sensor module according to a third embodiment of the present disclosure.
Figs. 3B-3C and Figs. 4B-4C are cross-sectional views of the sensor module according to the third embodiment of the present disclosure during the measurement.
Figs. 5A-5C are bottom views of the sensor module according to some embodiments of the present disclosure.
Fig. 6 shows a schematic diagram of an embodiment of a sensor module during the measurement.
Fig. 7 shows a vessel pulse signal, an electrocardiogram signal, and an aorta blood pressure signal of an object.

### DETAILED DESCRIPTION OF THE INVENTION

The following description is of the best-contemplated mode of carrying out the invention. This description is made for the purpose of illustrating the general principles of the invention and should not be taken in a limiting sense. For example, the formation of a first feature over, above, below, or on a second feature in the description that follows may include embodiments in which the first and second features are formed in direct contact, and may also include embodiments in which additional features may be formed between the first and second features, such that the first and second features may not be in direct contact. In addition, the present disclosure may repeat reference numerals and/or letters in the various examples. This repetition is for the purpose of simplicity and clarity and does not in itself dictate a relationship between the various embodiments and/or configurations discussed. The scope of the invention is best determined by reference to the appended claims.

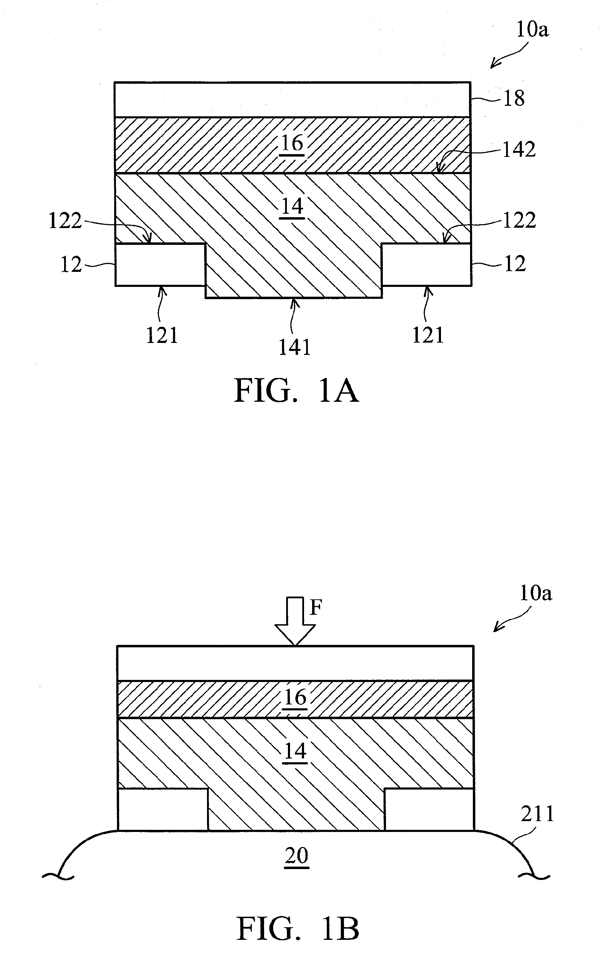

Fig. 1A is a cross section of a sensor module 10a according to a first embodiment of the present disclosure. The sensor module 10a for simultaneously measuring electrocardiography (ECG) and pulse signal of an object includes a first electrode 12, a second electrode 18 operatively connected to the first electrode 12, and a compressible material 16 and a deformable contact sensor 14 disposed between the first electrode 12 and the second electrode 18. The first electrode 12 having a first surface 121 and an opposite, second surface 122, a deformable contact sensor 14 having a first surface 141 and an opposite, second surface 142, wherein the first surfaces 121 and 141 of the first electrode 12 and the deformable contact sensor 14 are configured to face toward a region to be measured of the object. The compressible material 16 is disposed on the second surface 142 of the deformable contact sensor 14.

The first electrode 12 and the second electrode 18 used to receive the electrocardiography (ECG) signals may be made of a rigid, conductive material such as metal, electroplating plastics, or combinations thereof. The electrodes 12 and 14 can be cleaned or sterilized after the measurement, so that the sensor module 10a is reusable.

The deformable contact sensor 14 used to detect the pulse signal may include colloid, soft rubber, soft plastic, soft polymer, liquid silicone rubber, polydimethylsiloxane (PDMS), or combinations thereof. The thickness of the deformable contact sensor 14 may range from 1 mm to 10 mm, for example, from 8 mm to 10 mm. In one embodiment, the deformable contact sensor 14 is made of PDMS, having thickness of 8 mm. The deformable contact sensor 14 is incompressible, so that it is sensitive to the dynamic change of the measured region, such as pulsation vibration of an object.

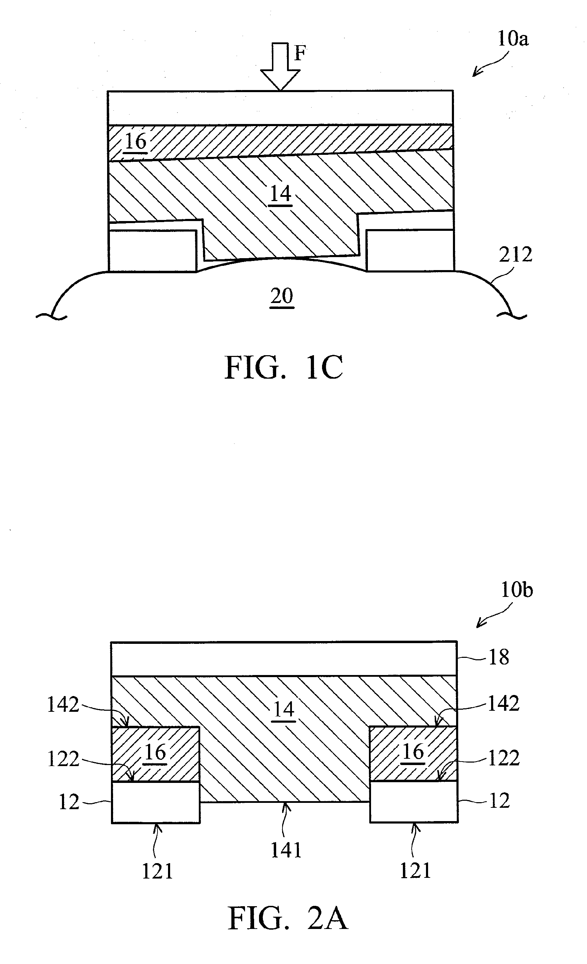

The compressible material 16 may include such material as foam, spring, air, or combinations thereof. In the first embodiment, the compressible material 16 is disposed on the second surface 142 of the deformable contact sensor 14, and the deformable contact sensor 14 is movable relative to the region to be measured of the object during the measurement, and the compressible material 16 may deform (or be compressed) accordingly to conform with the contour of the region to be measured of the object. It should be noted that, since there are different configurations of such as blood vessels or bones beneath the skin of the region to be measured, the region may have different contours. For example, Fig. 1B and Fig. 1C illustrate two exemplary embodiments of the cross-sectional views of the sensor module according to the first embodiment of the present disclosure during operation of the measurement. The region 20 to be measured may have a flat contour 211, as shown in Fig. 1B, or an irregular contour 212, as shown in Fig. 1C. The sensor module 10a is capable of self-adjusting to conform to the various contours due to the compressible material 16 between the deformable contact sensor 14 and the second electrode 18. A detailed description of the process of measurement according to the first embodiment is described below.

In Fig. 1B, the sensor module 10a is pressed by a force F to proceed with the measurement. The deformable contact sensor 14 moves upwards to conform to the contour 211 of the region 20 to be measured and the compressible material 16 is compressed evenly. Thus, the first electrode 12 and the deformable contact sensor 14 are both in contact with the region 20 of the object. Likewise, in Fig. 1C, the sensor module 10a is pressed by a force F to proceed with the measurement. However, in this case, the region 20 has an irregular contour 212. As shown in Fig. 1C, the deformable contact sensor 14 deforms and moves relative to the contour 212 of the region 20 of the object and the compressible material is compressed unevenly. Thereby, the first electrode 12 and the deformable contact sensor 14 are both in contact with the region 20 of the object.

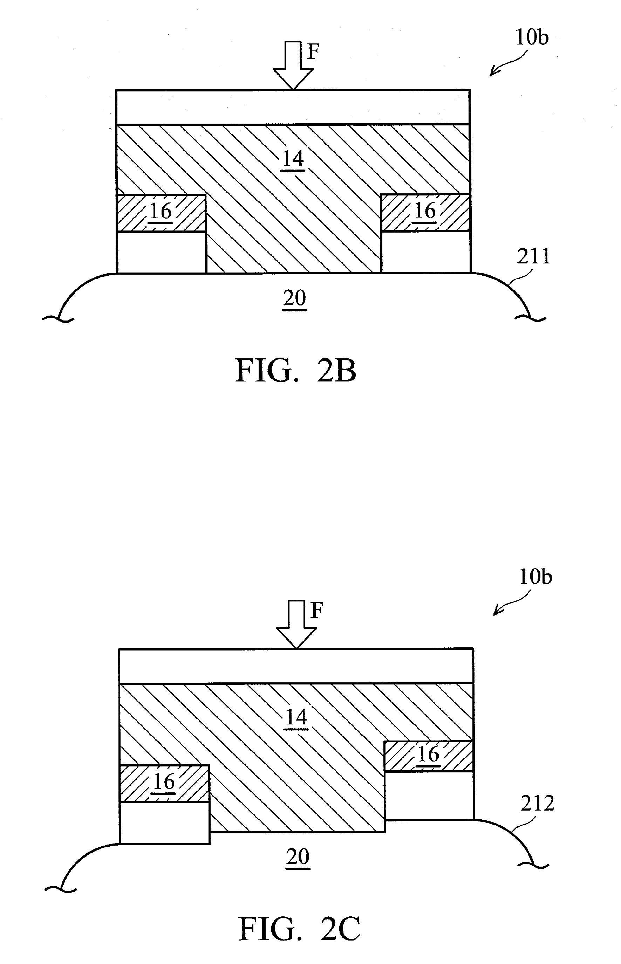

Fig. 2A is a cross section of a sensor module 10b according to a second embodiment of the present disclosure. The second embodiment differs from the first embodiment in that the compressible material 16 is disposed on the second surface 122 of the first electrode 12. Therefore, in the second embodiment, the first electrode 12 is movable relative to the region 20 to be measured of the object during the measurement. For example, Fig. 2B-2C are cross-sectional views of the sensor module according to the second embodiment of the present disclosure during operation of the measurement. Although the region 20 to be measured may have a flat contour 211, as shown in Fig. 2B, or an irregular contour 212, as shown in Fig. 2C, the sensor module 10b is capable of self-adjusting to conform to the various contours due to the compressible material 16 between the first electrode 12 and the deformable contact sensor 14. A detailed description of the process of measurement according to the second embodiment is described below.

In Fig. 2B, the sensor module 10b is pressed by a force F to proceed with the measurement. The first electrode 12 moves upwards to conform to the contour 211 of the region 20 to be measured and the compressible material 16 is compressed evenly. Thus, the first electrode 12 and the deformable contact sensor 14 are both in contact with the region 20 of the object. Likewise, in Fig. 2C, the sensor module 10b is pressed by a force F to proceed with the measurement. However, in this case, the region 20 has an irregular contour 212. As shown in Fig. 2C, the first electrode 12 moves relative to the contour 212 of the region 20 of the object and the compressible material is compressed unevenly. Thereby, the first electrode 12 and the deformable contact sensor 14 are both in contact with the region 20 of the object.

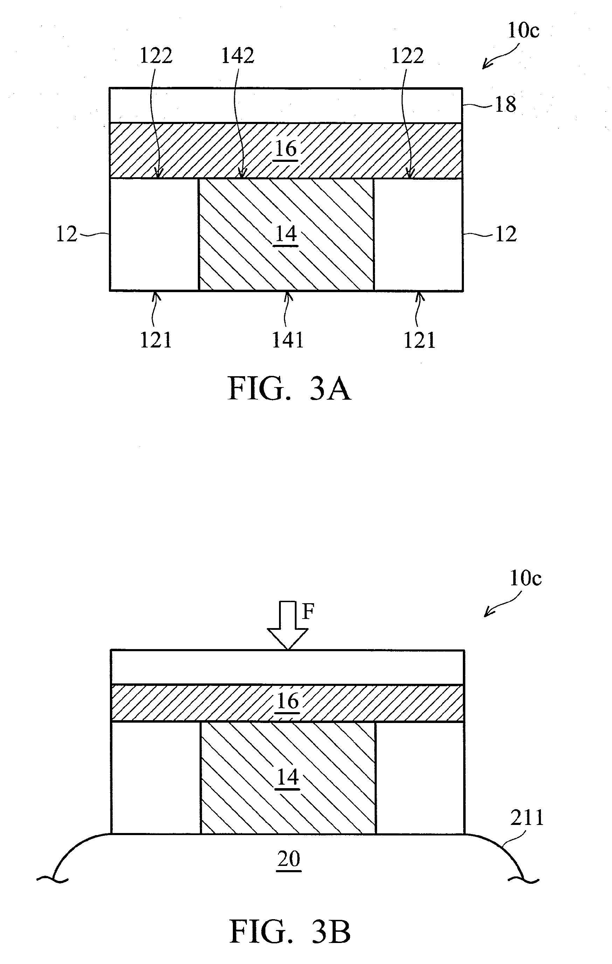

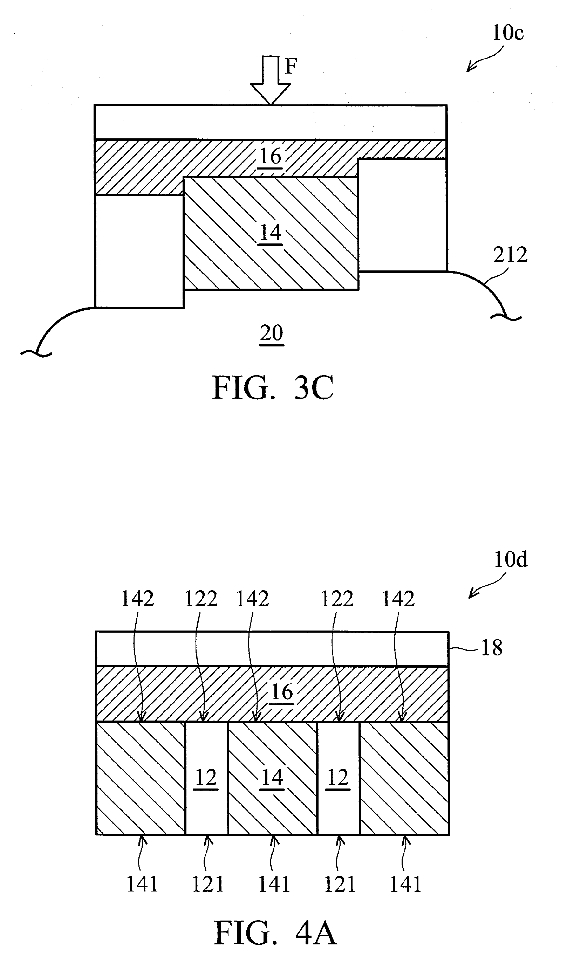

Fig. 3A and Fig. 4A are cross sections of a sensor module 10c and 10d according to a third embodiment of the present disclosure. The third embodiment differs from the first embodiment in that the compressible material 16 is disposed on both of the second surfaces 122 and 142 of the first electrode 12 and the deformable contact sensor 14. Therefore, in the third embodiment, both of the first electrode 12 and the deformable contact sensor 14 are movable relative to the region to be measured of the object during the measurement. In the third embodiment, various arrangements for the first electrode 12 and the deformable contact sensor 14 may be employed. For example, in some embodiments, the deformable contact sensor 14 may be embedded in the first electrode 12 as shown in Fig. 3A. In other embodiments, the first electrode 12 may be embedded in the deformable contact sensor 14, as shown in Fig. 4A. There may be at least one first electrode 12 embedded in the deformable contact sensor 14, for example. Although Figs. 4A-4C show two first electrodes 12 embedded in the deformable contact sensor 14, the number of the first electrode 12 is merely illustrative and should not be construed as a limitation.

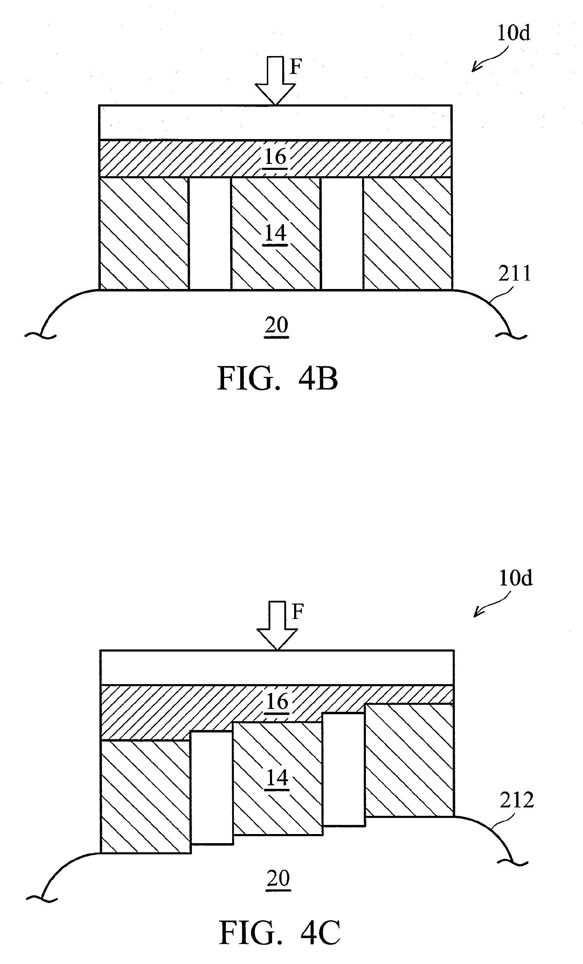

Figs. 3B-3C and Figs. 4B-4C are cross-sectional views of the sensor module according to the third embodiment of the present disclosure during operation of the measurement. Although the region 20 to be measured may have a flat contour 211, as shown in Fig. 3B and Fig. 4B, or an irregular contour 212, as shown un Fig. 3C and Fig. 4C, the sensor modules 10c and 10d are capable of self-adjusting to conform to the various contours of the region due to the compressible material 16 between the first electrode 12, the deformable contact sensor 14, and the second electrode 18. A detailed description of the process of measurement according to the third embodiment is described below.

In Fig. 3B, the sensor module 10c is pressed by a force F to proceed with the measurement. Both of the first electrode 12 and the deformable contact sensor 14 move upwards to conform to the contour 211 of the region 20 to be measured and the compressible material 16 is compressed evenly. Thus, the first electrode 12 and the deformable contact sensor 14 are both in contact with the region 20 of the object. Likewise, in Fig. 3C, the sensor module 10c is pressed by a force F to proceed with the measurement. However, in this case, the region 20 has an irregular contour 212. As shown in Fig. 3C, both of the first electrode 12 and the deformable contact sensor 14 move relative to the contour 212 of the region 20 of the object and the compressible material is compressed and deformed unevenly. Thereby, the first electrode 12 and the deformable contact sensor 14 are both in contact with the region 20 of the object.

In Fig. 4B, the sensor module 10d is pressed by a force F to proceed with the measurement. Both of the first electrodes 12 and the deformable contact sensor 14 move upwards to conform to the contour 211 of the region 20 to be measured and the compressible material 16 is compressed evenly. Thus, the first electrode 12 and the deformable contact sensor 14 are both in contact with the region 20 of the object. Likewise, in Fig. 4C, the sensor module 10d is pressed by a force F to proceed with the measurement. However, in this case, the region 20 has an irregular contour 212. As shown in Fig. 4C, both of the first electrode 12 and the deformable contact sensor 14 move relative to the contour 212 of the region 20 of the object and the compressible material is compressed and deformed unevenly. Thereby, the first electrode 12 and the deformable contact sensor 14 are both in contact with the region 20 of the object.

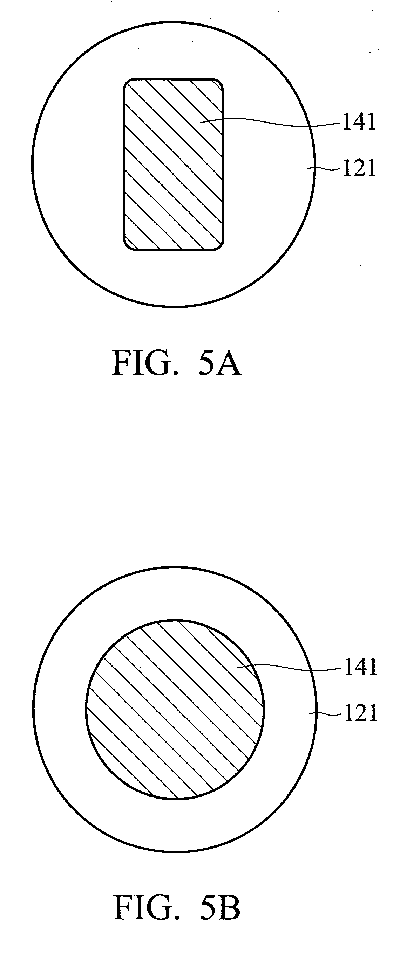

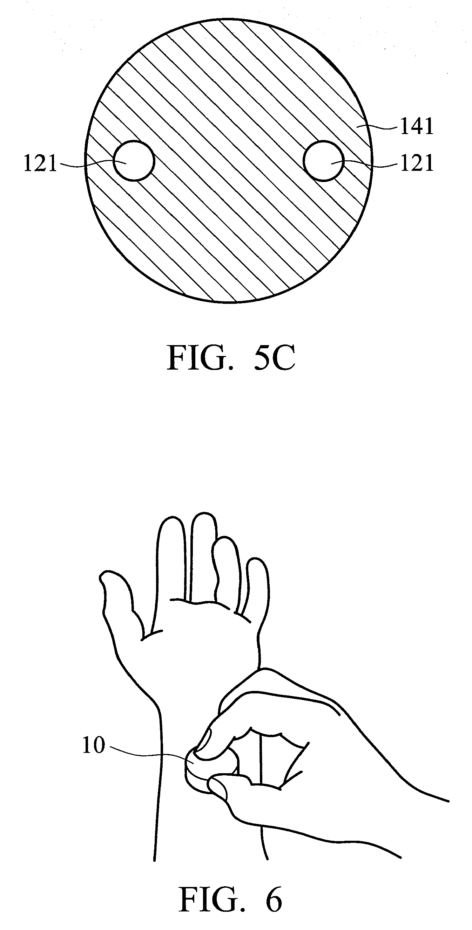

Figs. 5A-5C are bottom views of the sensor module according to some embodiments of the present disclosure. In one embodiment, the first surface 141 of the deformable contact sensor 14 may be rectangle and the first surface 121 of the first electrode 12 may be a rounded-shape, as shown in Fig. 5A. In another embodiment, the first surfaces 141 and 121 may be a rounded-shape, as shown in Fig. 5B. Fig 5A and Fig. 5B may be the bottom views of any one of the sensor modules 10a, 10b or 10c according to the first, second, or third embodiment of the present disclosure. Fig. 5C shows the bottom view of the sensor module 10d according to the third embodiment of the present disclosure. It should be realized that the first surface 141 of the deformable contact sensor 14 and the first surface 121 of the first electrode 12 may be any other suitable shape, such as a rounded-shape, oval, rectangle, square, rhombus, and so on, depending on the specific application needs.

The present disclosure also provides a method of using the sensor module 10. The method of using the sensor module 10 includes placing the sensor module 10 on the region to be measured of the object and positioning the sensor module 10 by a hand and touching the second electrode with at least one finger to measure the ECG and pulse signal simultaneously. Referring to Fig. 6, it shows the schematic diagram of an embodiment of a sensor module 10 during the measurement. In this embodiment, the user may place the sensor module 10 on the left wrist, position it by the right hand and press the top (the second electrode) of it with a finger. The region to be measured may be a position with pulsation and corresponding to a blood vessel, for example, an artery such as a radial artery or a carotid.

It should be realized that the region to be measured may be one of the right part and the left part of a body of the object, while the second electrode is contacted by a hand of the other of the right part or the left part of the body of the object. For example, the region to be measured may be such as a radial artery of left wrist or a carotid of left side of the neck. At this time, the user may use the right hand (right fingers) to position and press the sensor module on the left wrist or the left side of the neck. In another embodiment, the region to be measured may be such as a radial artery of right wrist or a carotid of right side of the neck. In such a case, the user may use the left hand (left fingers) to position and press the sensor module on the right wrist or the right side of the neck.

During the measurement, the touching of the user not only fixes the sensor module but also makes both of the first electrodes and the deformable contact sensor of the sensor module firmly in contact with the skin of the region to be measured of the object. Thus, the sensor module may detect the ECG and pulse signals simultaneously. By the described method of using the sensor module 10, the user may have the sensor module 10 on-set more easily. As described previously, since there are different configurations of such as blood vessels or bones beneath the skin of the region to be measured, the region may have different contours. However, as shown in Figs. 1B-1C, 2B-2C, 3B-3C, and 4B-4C, since the first electrodes 12 and/or deformable contact sensor 14 move relative to the region 20 to be measured of the object and accompanied deformation (or compression) of the compressible material 16, the sensor module finally conform to the contour 211/212 of the region 20.

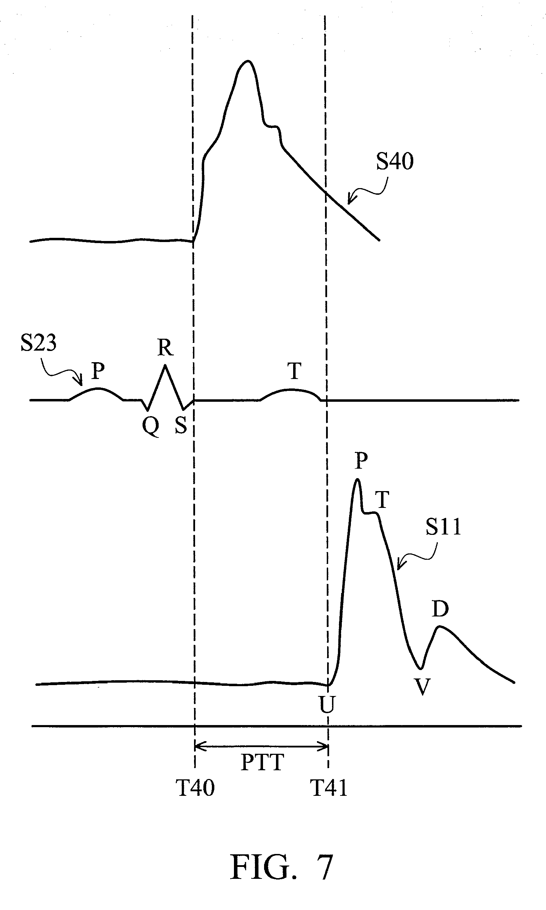

The present disclosure also provides use of the described sensor module to measure pulse transition time (PTT) and derived indices thereof. Pulse transition time (PTT) indicates the time period when the pressure wave of the blood pressure is output to the region to be measured of the object from the heart of the object. By calculating an arrival time difference between the ECG and the pulse signal, the pulse transition time (PTT) may be obtained. FIG. 7 shows the vessel pulse signal S11, the electrocardiogram signal S23, and an aorta blood pressure signal S40 of the object. When the aortic valve of the heart of the object is open, the blood flows to the aorta from the heart. At this time, the blood pressure of the aorta starts rising. The blood pressure of the limbs starts rising after a delayed time period. Referring to FIG. 4, the signal S40 starts rising at a time point T40. Moreover, the time point T41 when a rising waveform starts appearing on the vessel pulse signal S11 is used to serve as the other reference point. The rising waveform indicates that the blood pressure of the region to be measured rises. By calculating the difference between the reference time points T41 and T40, the pulse transmission time (PTT) is obtained.

Moreover, pulse transmission time (PTT) may be used to calculate the pulse wave velocity (PWV), which serves as an index of risk possibility of arterial stiffness, by obtaining the distance between the region to be measured and the heart of the object. In addition, derived indices of pulse transition time (PTT) may include blood pressure (BP).

The designation of the sensor module makes the users have the sensor module on-set easily by themselves. By integrating the ECG electrodes and pulse sensor in a module, the two in one sensor module of the present disclosure may contact the same region of the body of the object. Thus, the users may easily position the sensor module on the region to be measured by a hand and touching it with finger. Thereby, both of the ECG electrodes and pulse sensor may touch the skin firmly and the ECG and pulse signals may be measured simultaneously. The tedious procedure of contacting the sensor to the correct position and fastening the sensor by a strap or stuck in order to attach ECG electrodes may be mitigated.

While the invention has been described by way of example and in terms of the preferred embodiments, it is to be understood that the invention is not limited to the disclosed embodiments. On the contrary, it is intended to cover various modifications and similar arrangements (as would be apparent to those skilled in the art). Therefore, the scope of the appended claims should be accorded the broadest interpretation so as to encompass all such modifications and similar arrangements.

## Claims

1. A sensor module (10a-d, 10) for simultaneously measuring electrocardiography (ECG) and pulse signal of an object, comprising:
a first electrode (12) having a first surface (121) and an opposite, second surface (122);
a deformable contact sensor (14) having a first surface (141) and an opposite, second surface (142), wherein the first surfaces (121, 141) of the first electrode (12) and the deformable contact sensor (14) are configured to face toward a region (20) to be measured of the object;
a compressible material (16) disposed on at least one of the second surfaces (122, 142) of the first electrode (12) and the deformable contact sensor (14); and
a second electrode (18) operatively connected to the first electrode (12).

2. The sensor module (10a-d, 10) as claimed in claim 1, wherein the compressible material (16) is disposed on the second surface (122) of the first electrode (12).

3. The sensor module (10a-d, 10) as claimed in claim 1, wherein the compressible material (16) is disposed on the second surface (142) of the deformable contact sensor (14).

4. The sensor module (10a-d, 10) as claimed in claim 1, wherein the compressible material (16) is disposed on both of the second surfaces (122, 142) of the first electrode (12) and the deformable contact sensor (14).

5. The sensor module (10a-d, 10) as claimed in claim 4, wherein the deformable contact sensor (14) is embedded in the first electrode (12).

6. The sensor module (10a-d, 10) as claimed in claim 4, wherein the first electrode (12) is embedded in the deformable contact sensor (14).

7. The sensor module (10a-d, 10) as claimed in one of the preceding claims, wherein the second electrode (18) is disposed opposite to the first electrode (12).

8. The sensor module (10a-d, 10) as claimed in one of the preceding claims, wherein both the first surfaces (121, 141) of the first electrode (12) and the deformable contact sensor (14) are movable to conform with a contour (211, 212) of the region (20) to be measured of the object.

9. A method of using the sensor module (10a-d, 10) as set forth in claim 1, comprising placing the sensor module (10a-d, 10) on the region (20) to be measured of the object and positioning the sensor module (10a-d, 10) by a hand and touching the second electrode (18) with at least one finger to measure the ECG and pulse signal simultaneously.

10. A method as claimed in claim 9, wherein both the first surfaces (121, 141) of the first electrode (12) and the deformable contact sensor (14) are in contact with the region (20) to be measured of the object simultaneously during the measurement.

11. A method as claimed in claim 9, wherein both the first surfaces (121, 141) of the first electrode (12) and the deformable contact sensor (14) are movable to conform with a contour (211, 212) of the region (20) to be measured of the object.

12. Use of a sensor module (10a-d, 10) as set forth in claim 1 to measure pulse transition time (PTT) and derived indices thereof by calculating an arrival time difference between the ECG and the pulse signal.

13. The use as claimed in claim 12, wherein the derived indices comprise pulse wave velocity (PWV) or blood pressure (BP).

14. The use as claimed in claim 12 or 13, wherein the sensor module (10a-d, 10) is placed on the region (20) to be measured of the object by a hand with a finger giving a force (F) to the second electrode (18) to measure ECG and pulse signal simultaneously.

15. The use as claimed in one of claims 12 to 14, wherein both the first surfaces (121, 141) of the first electrode (12) and the deformable contact sensor (14) are attached to the region (20) to be measured of the object simultaneously.
